# EUROPÄISCHE PATENTANMELDUNG

(11) **EP 0 531 719 A1**
(43) Veröffentlichungstag der Anmeldung: **17.03.1993**
(21) Anmeldenummer: 92113530.7
(22) Anmeldetag: 08.08.1992
(51) Int. Cl.: C07D 209/50, G03G 9/08

(54) **Elektrofotografische Toner und 3-Iminoisoindolinone**

(30) Priorität: 23.08.1991 DE 4128080
(71) Anmelder: BAYER AG, 51368 Leverkusen (DE)
(72) Erfinder: Köcher, Matthias, Dr., W-5063 Overath (DE); Raue, Roderich, Dr., W-5090 Leverkusen 1 (DE); Wunderlich, Klaus, Dr., W-5090 Leverkusen 1 (DE)

(57) **Zusammenfassung**

Besonders vorteilhafte elektrofotografische Toner enthalten neben üblichen Harz- und Pigmentpartikeln ein die negative Ladung verstärkendes Additiv der allgemeinen Formeln 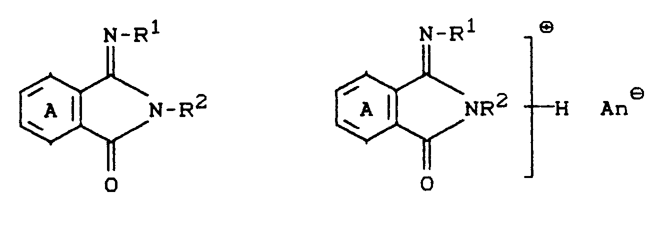
in denen
einer der Reste R¹ und R² für Wasserstoff und der andere dieser Reste für Wasserstoff, C₁-C₂₂-Alkyl, Allyl, Cyclohexyl, Phenyl-C₁-C₂-alkyl oder Phenyl steht,
An^{⊖} ein Anion bedeutet,
der Ring A und/oder die Reste R¹ oder R² substituiert sein können und
der an der Klammer bei Formel (II) stehende Wasserstoff an ein Stickstoffatom gebunden ist,
und/oder eine diesen Formeln (I) und/oder (II) entsprechende tautomere Verbindung.

## Beschreibung

Die vorliegende Erfindung betrifft elektrofotografische Toner, die übliche Harz- und Pigmentpartikel enthalten und dadurch gekennzeichnet sind, daß sie ein die negative Ladung verstärkendes Additiv der allgemeinen Formeln (I) und/oder (II) enthalten 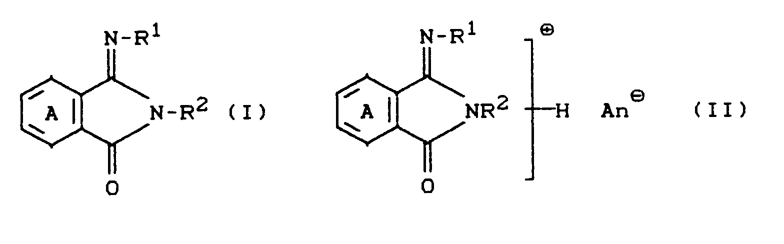
in denen
einer der Reste R¹ und R² für Wasserstoff und der andere dieser Reste für Wasserstoff, C₁-C₂₂-Alkyl, Allyl, Cyclohexyl, Phenyl-C₁-C₂-alkyl oder Phenyl steht,
An^{⊖} ein Anion bedeutet,
der Ring A und/oder die Reste R¹ oder R² substituiert sein können und
der an der Klammer bei Formel (II) stehende Wasserstoff an ein Stickstoffatom gebunden ist,
und/oder eine den Formeln (I) und/oder (II) entsprechende tautomere Verbindung enthalten.

Bevorzugt sind Verbindungen der Formeln (I) und/oder (II), in denen
einer der Reste R¹ und R² für Wasserstoff und der andere dieser Reste für Wasserstoff, gegebenenfalls substituiertes C₁-C₁₈-Alkyl, gegebenenfalls substituiertes Benzyl oder gegebenenfalls substituiertes Cyclohexyl steht.

Bei den Substituenten für den Ring A und für C₁-C₁₈-Alkyl, Benzyl und Cyclohexyl in R¹ und R² kann es sich z.B. um C₁-C₄-Alkyl, C₁-C₄-Alkoxy, Hydroxy, Halogen wie Fluor, Chlor und Brom, Cyan, einen Carbamoyl- oder Sulfamoylrest, der gegebenenfalls durch 1 bis 2 C₁-C₄-Alkylreste substituiert sein kann, C₁-C₄-Alkoxycarbonyl oder Phenyl handeln, bei den Substituenten für C₁-C₁₈-Alkyl, Benzyl und Cyclohexyl in R¹ und R² aber auch um Carboxy-, Sulfo- oder Sulfatogruppen, die Verbindungen der Formel (I) zur Bildung eines inneren Salzes befähigen.

Bevorzugte Substituenten an den C₁- bis C₁₈-Alkylresten sind Hydroxy, C₁-C₄-Alkoxy, Chlor, Cyan, Carbamoyl, C₁-C₂-Alkoxycarbonyl, Carboxy, Sulfo und Sulfato.

Bei An^{⊖} kann es sich um ein übliches Anion handeln, beispielsweise um Halogenid wie Chlorid, Bromid und Iodid, Tetrafluoroborat oder um ein Anion von Alkyl- und Arylsulfonsäuren, Alkyl- und Arylcarbonsäuren, Alkyl- und Arylphosphorsäuren und Alkyl- und Arylphosphonsäuren handeln, Bevorzugt sind Anionen, die die Wasserlöslichkeit der Verbindungen der Formel (II) erniedrigen wie Arylsulfonsäure- und Arylcarbonsäurereste, insbesondere Tosylat-, Benzoat- und Phthalatreste. Die Verminderung der Wasserlöslichkeit kann aber auch dadurch erfolgen, daß man einen relativ großen Alkylrest R¹ oder R² wählt, beispielsweise im Bereich C₈-C₂₂-Alkyl. In diesem Falle sind auch relativ hydrophile Anionen, z.B. Halogenide gut geeignet. An^{⊖} kann aber auch Bestandteil des Restes R¹ oder R² sein, bei dem es sich in diesem Fall dann beispielsweise um eine Sulfoalkyl-, Sulfatoalkyl- oder Carboxyalkylgruppe in Form ihres Anions handeln kann.

Von den Verbindungen der Formel (II) sind solche bevorzugt, deren Wasserlöslichkeit bei 20°C unter 3 Gew.-%, insbesondere unter 1 Gew.-% beträgt. An^{⊖} steht vorzugsweise für ein Halogenid, Tetrafluoroborat, Arylsulfonat, gegebenenfalls durch C₁-C₁₂-Alkyl oder Chlor substituiertes Benzolsulfonat, C₁-C₁₈-Alkylsulfonat, C₂-C₁₀-Perfluoralkylsulfonat, das Anion einer C₂-C₁₈-Alkylcarbonsäure, das Anion eines Kondensationsprodukts aus Formaldehyd und einer Arylsulfonsäure, sulfoniertes 4,4'-Dihydroxydiphenylsulfon, sowie ein Anion einer Heteropolysäure auf der Basis Wolfram und/oder Molybdän mit Phosphor und/oder Silicium, insbesondere ein Anion einer Phosphorwolframmolybdatsäure oder einen Rest derart, wie er bereits bei R¹ und R² als anionischer Rest beschrieben ist.

Die Verbindungen der Formel (II) können z.T. nach an sich bekannten Methoden hergestellt werden, indem man Verbindungen der Formel (I) und/oder eine dazu tautomere Verbindung mit einer Verbindung der Formel H^{⊕}An^{⊖}, in der An^{⊖} die oben angegebene Bedeutung hat, umsetzt, und gegebenenfalls anschließend das eingeführte Anion gegen ein anderes austauscht.

Die Reaktion wird im allgemeinen in einem organischen Lösungsmittel und im Temperaturbereich von 10 bis 200°C, vorzugsweise bei 20 bis 140°C durchgeführt. In Einzelfällen ist auch Wasser als Lösungsmittel geeignet. Das Reaktionsprodukt der Formel (II) kristallisiert in der Regel aus der Reaktionslösung aus und kann daraus z.B. durch Abfiltrieren isoliert werden. Man kann die Lösung aber auch eindampfen und das Reaktionsprodukt der Formel (II) auf diese Weise als kristallines Pulver gewinnen.

Verbindungen der Formel (I) mit R¹ oder R² ungleich Wasserstoff kann man beispielsweise herstellen, indem man eine Verbindung der Formel (III) 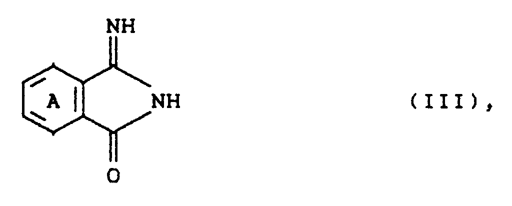
in der der Ring A gegebenenfalls wie bei den Formeln (I) und (II) angegeben substituiert sein kann,
mit einem primären Amin der Formeln R¹-NH₂ oder R²-NH₂, in denen R¹ und R² eine bei den Formeln (I) und (II) angegebene, von Wasserstoff verschiedene Bedeutung hat, unter Abspaltung von Ammoniak kondensiert.

Diese Reaktion wird im allgemeinen in einem organischen Lösungsmittel und im Temperaturbereich von 0 bis 150°C, vorzugsweise bei 20 bis 80°C durchgeführt. In Einzelfällen, insbesondere wenn R¹ oder R² für einen Sulfo- oder Sulfatogruppen enthaltenden Rest steht, ist auch Wasser als Lösungsmittel geeignet.

Eine besondere, erfinderische Variante zur Herstellung von Verbindungen der Formel (II), in denen sich der anionische Rest am Substituenten R¹ oder R² befindet, besteht in der direkten Umsetzung einer Verbindung der Formel (III) mit einem entsprechend substituierten Amin, z.B. mit einer Aminoalkancarbonsäure, Aminoalkylsulfonsaure oder einem Sulfatoalkylamin.

Eine weitere Variante zur Herstellung derartiger Verbindungen besteht in der nachträglichen Einführung des anionischen Restes in den Substituenten R¹, z.B. durch Überführung einer Hydroxyalkylgruppe in eine Sulfatoalkylgruppe.

Zu Verbindungen der Formel (II), in denen R¹ und R² für Wasserstoff stehen, kann man in erfinderischer Weise gelangen, wenn man ein Isoindolin der Formel (IV) 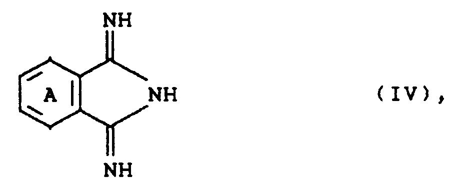
in der der Ring A gegebenenfalls wie bei den Formeln (I) und (II) angegeben substituiert sein kann
mit einer Verbindung der Formel H^{⊕}An^{⊖}, worin An^{⊖} die oben angegebene Bedeutung hat, in einem Alkanol, vorzugsweise Methanol, umsetzt. Aus den so erhältlichen Verbindungen der Formel (II) kann man beispielsweise durch Behandlung mit wäßriger Sodalösung Verbindungen der Formel (I) mit R¹ = R² = Wasserstoff herstellen.

Geeignete organische Lösungsmittel sind beispielsweise Sulfolan, Aromaten wie Toluol, Chlorbenzol, o-Dichlorbenzol, Trichlorbenzole und Xylole, Alkanole wie Methanol, Ethanol, Propanol, Isopropanol, n-Butanol, 2-Methoxyethanol und 2-Ethoxyethanol, Alkandiole wie Ethylenglykol, Dialkoxyalkane wie Ethylenglykoldimethylether, Nitrile wie Acetonitril, chlorierte Aliphaten wie Methylenchlorid und Chloroform und dipolar aprotische Lösungsmittel wie Dimethylformamid, N-Methylpyrrolidon oder Dimethylsulfoxid.

Die Verbindungen der Formeln (I) und (II) sind zumeist farblos oder nur schwach gefärbt. Verbindungen der Formeln (I) und (II), in denen R¹ für Phenyl steht, haben meistens eine gelbliche Eigenfarbe.

Toner finden Verwendung in der Entwicklung elektrostatischer Ladungsbilder in der Elektrofotografie, Elektrografie und Ionografie, sowie magnetostatischer Ladungsbilder in der Magnetografie. Von diesen Verfahren hat die Elektrofotografie (siehe z.B. US-PS 2 297 691, US-PS 3 666 363 und US-PS 4 071 361) die mit Abstand größte Bedeutung erlangt.

Dabei einsetzbare Toner erhält man z.B. nach Einkneten von ca. 1 bis 3 Gew.-% Ladungskontrollsubstanzen, ca. 2 bis 5 Gew.-% Pigmenten und gegebenenfalls weiteren Additiven (z.B. 1 bis 3 Gew.-% Polyolefinwachse zur Steigerung der adhäsiven Eigenschaften) in das Basisharz, anschließendem feinem Mahlen, nachfolgender Einstellung einer definierten Teilchengröße durch Sichtung und gegebenenfalls abschließender Einstellung von gewünschten Pulverfließeigenschaften durch Zusatz von feinteiliger Kieselsäure oder ähnlichen Produkten. Geeignete Basisharze sind beispielsweise Styrol/Acrylat-, Styrol/Methacrylat- und Styrol/Butadien-Copolymere und Polyester.

Erfindungsgemäße Toner zeichnen sich dadurch aus, daß sie als Ladungskontrollsubstanzen Verbindungen der Formeln (I) und/oder (II) und/oder dazu tautomere Verbindungen enthalten.

Für die Qualität des Toners ist die Einheitlichkeit der einzelnen Tonerpartikel von größter Wichtigkeit.

Daher werden höchste Anforderungen an die Dispergierbarkeit der Additive, speziell der Ladungskontrollsubstanzen gestellt.

Die verwendeten Additive, speziell die Ladungskontrollsubstanzen, dürfen keine chemischen Wechselwirkungen mit Geräteteilen (z.B. Fotoleitern und Fixierwalzen) eingehen, welche zu irreversiblen Verschmutzungen und Beschädigungen der betroffenen Geräteteile führen würden.

Hierbei ist insbesondere im Hinblick auf die beheizten Fixierwalzen eine hohe Thermostabilität der Ladungskontrollsubstanzen erforderlich.

Zusätzlich zu diesen vielfältigen Anforderungen an Ladungskontrollsubstanzen dürfen bei farbigen Fotokopien und anderen farbigen Bildreproduktionen die Additive keine oder nur eine geringe Eigenfarbe aufweisen.

Außerdem zeichnen sich die erfindungsgemäßen Ladungskontrollsubstanzen durch hohe Thermostabilität aus, so daß bei den üblichen Verarbeitungstemperaturen von ca. 250°C während des Extrusionsvorgangs keine Zersetzungserscheinungen auftreten, wie sie häufig bei den üblicherweise verwendeten Metallkomplexen beobachtet werden Schließlich zeichnen sich die erfindungsgemäßen Ladungskontrollsubstanzen auch durch eine gute Umweltverträglichkeit aus, da sie völlig metallfrei sind und daher im Gegensatz zu den auf Metallkomplexen basierenden üblichen Ladungskontrollsubstanzen beim Recycling des damit versehenen Papiers keine Kontamination durch Metalle erfolgen kann.

Erfindungsgemäße Toner zeichnen sich durch eine hohe negative Aufladung, eine gute Langzeitstabilität und weitgehende Unempfindlichkeit gegenüber Luftfeuchtigkeit aus.

Die vorliegende Erfindung betrifft weiterhin neue 3-Iminoisoindolinone der Formel (I') 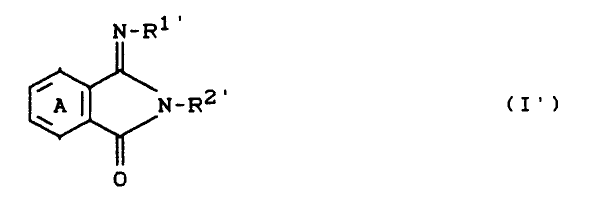
in der
einer der Reste R^{1'} und R^{2'} für Wasserstoff und der andere dieser Reste für Wasserstoff oder eine C₁-C₂₂-Alkylgruppe steht, die durch eine oder mehrere Carboxy-, Sulfo- und/oder Sulfatogruppen substituiert ist.

Bevorzugte 3-Iminoisoindolinone der Formel (I') sind

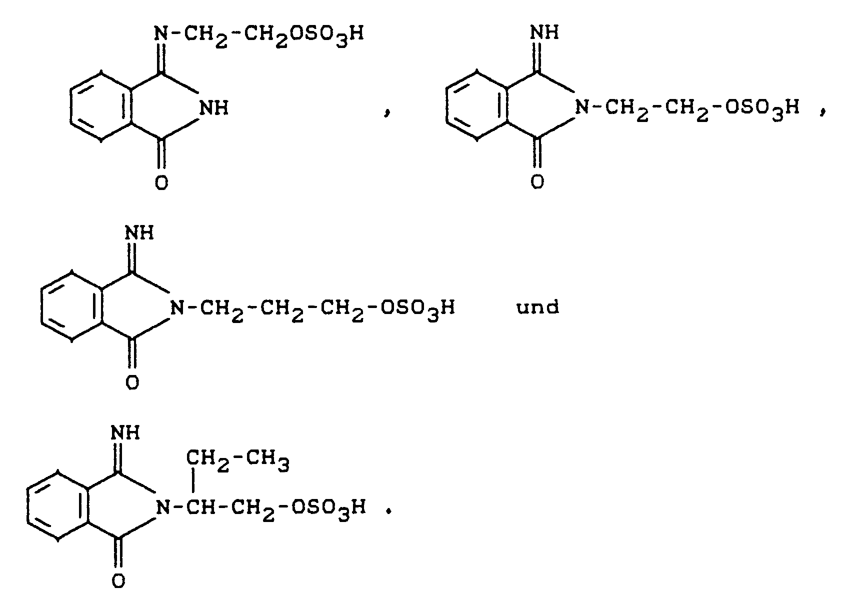

Die Herstellung und Anwendung von 3-Iminoisoindolinonen der Formel (I') ist bereits weiter oben beschrieben worden.

### Beispiele

### Beispiel 1

Eine Lösung von 342 g p-Toluolsulfonsäure in 360 ml Toluol wurde auf 10°C abgekühlt und mit einer Lösung von 14,5 g 3-Amino-1-imino-isoindolenin in 600 ml Methanol versetzt. Die Temperatur stieg auf 33°C an. Nach kurzer Zeit trat eine kristalline Fällung auf. Es wurde 2,5 Stunden bei Raumtemperatur nachgerührt, abgesaugt und der Rückstand mit 1000 ml Methanol gründlich gewaschen. Nach dem Trocknen des Rückstandes bei 70°C im Vakuum wurden 156,1 g einer Verbindung der Formel (II) mit R¹ = R² = Wasserstoff und An^{⊖} = p-Toluolsulfonat erhalten.

| Elementaranalyse: | C | H | N | O | S |
|---|---|---|---|---|---|
| ber.: | 56,8 | 4,7 | 8,8 | 20,1 | 10,1 % |
| gef.: | 56,6 | 4,6 | 8,7 | 19,8 | 10,1 % |

### Beispiel 2

250 g der nach Beispiel 1 erhaltenen Verbindung wurden in 2500 ml Wasser eingerührt und langsam mit 250 ml 20 gew.-%iger Sodalösung versetzt. Der pH-Wert betrug nach Beendigung des Eintropfens 8,5. Es wurde 7 Stunden bei Raumtemperatur nachgerührt, anschließend abgesaugt, mit 1000 ml Wasser gewaschen und bei 60°C im Vakuum getrocknet. Erhalten wurden 86,2 g 3-Iminoisoindolinon (Formel (I), R¹ = R² = Wasserstoff).
Schmelzpunkt: 207 bis 208°C

| Elementaranalyse: | C | H | N | O |
|---|---|---|---|---|
| ber.: | 65,7 | 4,1 | 19,2 | 11,0 % |
| gef.: | 65,8 | 4,2 | 19,2 | 11,2 % |

### Beispiel 3

192 g des nach Beispiel 2 erhaltenen 3-Iminoisoindolinons wurden mit 1280 ml Methanol angerührt und mit 189,4 g Stearylamin (90 gew.-%ig) versetzt. Nach 2-stündigem Rühren bei 40°C wurde eine Lösung von 228 g p-Toluolsulfonsäure in 200 ml Methanol zugegeben und 2 Stunden bei 40°C nachgerührt. Nach 1-stündigem Erwärmen auf 60°C wurde noch 10 Stunden bei Raumtemperatur nachgerührt. Anschließend wurde abgesaugt, mit 600 ml Methanol gewaschen und bei 60°C im Vakuum getrocknet. Erhalten wurden 245 g einer Verbindung der Formel (II) mit R¹ = H, R² = C₁₈H₃₇ und An^{⊖} = p-Toluolsulfonat.
Schmelzpunkt: 134 bis 146°C (unter Zersetzung)

| Elementaranalyse; | C | H | N | O | S |
|---|---|---|---|---|---|
| ber.: | 69,5 | 8,8 | 4,9 | 11,2 | 5,6 % |
| gef.: | 69,2 | 8,4 | 4,9 | 11,2 | 5,7 % |

### Beispiel 4

160 g des nach Beispiel 2 erhaltenen 3-Iminoisoindolinons wurden in 600 ml Methanol angerührt und mit 74,3 g Cyclohexylamin versetzt. Nach 3-stündigem Rühren bei 40°C wurden 285 g p-Toluolsulfonsäure zugegeben und 1 Stunde bei 60°C nachgerührt. Anschließend wurden langsam 1200 ml Wasser zugefügt und noch 10 Stunden bei Raumtemperatur gerührt. Dann wurde abgesaugt, mit 1500 ml Wasser gewaschen und bei 60°C im Vakuum getrocknet. Erhalten wurden 114 g einer Verbindung der Formel (II) mit R¹ = Cyclohexyl, R² = H und An^{⊖} = p-Toluolsulfonat.

| Elementaranalyse: | C | H | N | O | S |
|---|---|---|---|---|---|
| ber.: | 63,0 | 6,0 | 7,0 | 16,0 | 8,0 % |
| gef.: | 63,2 | 5,9 | 6,9 | 15,9 | 7,9 % |

### Beispiel 5

146 g des nach Beispiel 2 erhaltenen 3-Iminoisodinolinons wurden in 600 ml Methanol angerührt und mit 73,2 g 2-Aminoethanol versetzt. Nach 2-stündigem Rühren bei 45°C wurde erkalten gelassen, abgesaugt, mit 300 ml Methanol gewaschen und im Vakuum bei 60°C getrocknet. Erhalten wurden 143 g einer Verbindung der Formel (I) mit R¹ = H und R² = Hydroxyethyl.

| Elementaranalyse: | C | H | N | O |
|---|---|---|---|---|
| ber.: | 63,2 | 5,3 | 14,7 | 16,8 % |
| gef.: | 63,0 | 5,7 | 14,6 | 17,0 % |

### Beispiel 6

190 g des nach Beispiel 5 erhaltenen 3-Imino-2-(2-hydroxyethyl)-isoindolinons wurden in 2000 ml Aceton eingerührt und bei 40°C mit einer Lösung von 380 g p-Toluolsulfonsäure in 500 ml Aceton versetzt. Nach dem Abkühlen auf Raumtemperatur und 2-stündigem Nachrühren wurde abgesaugt, mit 1000 ml Aceton gewaschen und bei 60°C getrocknet. Es wurden 278 g einer Verbindung der Formel (II) mit R¹ = H, R² = 2-Hydroxyethyl und An^{⊖} = p-Toluolsulfonat erhalten.

| Elementaranalyse: | C | H | N | O | S |
|---|---|---|---|---|---|
| ber.: | 56,4 | 5,0 | 7,7 | 22,1 | 8,8 % |
| gef.: | 55,8 | 5,1 | 7,6 | 22,2 | 8,8 % |

Schmelzpunkt: 154 bis 156°C

### Beispiel 7

65,7 g des nach Beispiel 2 erhaltenen 3-Iminoisoindolinons wurden in 450 ml Methanol angerührt und mit 162,0 g Perfluorbutansulfonsäure versetzt. Hierbei stieg die Temperatur auf 55°C an. Nach 10-stündigem Rühren bei Raumtemperatur wurde das Methanol im Vakuum abdestilliert, der Rückstand mit 300 ml Wasser verrührt, abgesaugt, mit 300 ml 10 gew.-%iger wäßriger Natriumchloridlösung und anschließend mit 100 ml Wasser gewaschen. Nach dem Trocknen wurden 117,2 g einer Verbindung der Formel (II) mit R¹ = R² = Wasserstoff und An^{⊖} = Perfluorbutansulfonat erhalten.

| Elementaranalyse: | C | H | N | F | S |
|---|---|---|---|---|---|
| ber.: | 32,3 | 1,5 | 6,3 | 38,3 | 7,2 % |
| gef.: | 34,0 | 2,5 | 6,0 | 34,5 | 6,6 % |

### Beispiel 8

43,8 g des nach Beispiel 2 erhaltenen 3-Iminoisoindolinons wurden in 600 ml Wasser eingerührt und mit 56,6 g Sulfatoethylamin versetzt. Anschließend wurde mit 10 gew.-%iger wäßriger Natronlauge (ca. 25 ml) der pH-Wert auf 8,5 eingestellt und 20 Stunden unter Rühren bei diesem Wert gehalten. Es wurde durch Filtration geklärt und das Filtrat mit 28 gew.-%iger wäßriger Salzsäure ein pH-Wert von 1,5 eingestellt. Nach 2-stündigem Rühren wurde die kristalline Fällung abgesaugt und mit Wasser neutral gewaschen. Nach dem Trocknen bei 60°C wurden 67,7 g einer Verbindung der Formel (I) mit R¹ = H und R² = -CH₂-CH₂-OSO₃H oder der Formel 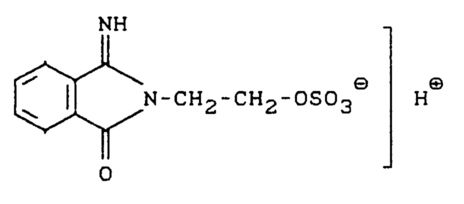
erhalten.

| Elementaranalyse: | C | H | N | O | S |
|---|---|---|---|---|---|
| ber.: | 44,4 | 3,7 | 10,4 | 29,6 | 11,9 % |
| gef.: | 43,7 | 4,3 | 9,9 | 31,3 | 11,0 % |

Schmelzpunkt: 253 bis 255°C (unter Zersetzung)

### Beispiel 9

429 g einer 17,5 gew.-%igen wäßrigen Taurin-Lösung wurden in 900 ml Wasser eingerührt und mit konzentrierter Salzsäure auf einen pH-Wert von 9,5 eingestellt. Anschließend wurden 87,6 g 3-Iminoisoindolinon (erhalten nach Beispiel 2) hinzugefügt. Nach 10 Stunden Rühren bei Raumtemperatur und einem pH-Wert von 9,5 wurde durch Filtration geklärt und das Filtrat mit Salzsäure auf einen pH-Wert von 1,0 eingestellt. Es wurde noch 1 Stunde bei 10°C gerührt, dann wurde der kristalline Niederschlag abgesaugt und mit 500 ml Eiswasser neutral gewaschen. Nach dem Trocknen bei 60°C wurden 135,6 g einer Verbindung der Formel (I) mit R¹ = H und R² = -CH₂-CH₂-SO₃H oder der Formel 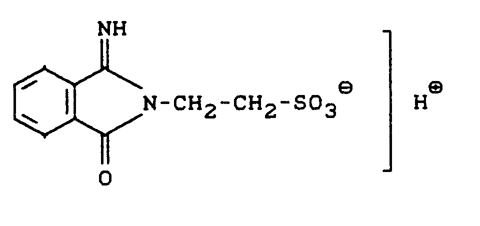
erhalten.

| Elementaranalyse: | C | H | N | O | S |
|---|---|---|---|---|---|
| ber.: | 47,2 | 3,9 | 11,0 | 25,2 | 12,6 % |
| gef.: | 47,2 | 4,2 | 11,0 | 25,8 | 12,6 % |

### Beispiel 10

38,0 g des nach Beispiel 5 erhaltenen 3-(Hydroxyethylimino)-isoindolinons wurden unter Kühlung bei 10 bis 20°C in 100 ml Schwefelsäuremonohydrat eingetragen. Nach 3 Stunden Rühren bei Raumtemperatur wurde auf 400 ml Eiswasser ausgetragen. Die kristalline Fällung wurde abgesaugt, mit 600 ml Eiswasser neutral gewaschen und bei 60°C getrocknet. Erhalten wurden 54,0 g eines Produktes, das mit dem des Beispiels 8 identisch war.

| Elementaranalyse: | C | H | N | O | S |
|---|---|---|---|---|---|
| ber.: | 44,4 | 3,7 | 10,4 | 29,6 | 11,9 % |
| gef.: | 42,6 | 3,8 | 10,0 | 31,5 | 12,4 % |

### Beispiel 11

146,0 g 3-Iminoisoindolinon (erhalten nach Beispiel 2) wurden in 1500 ml Wasser eingerührt und mit 111,3 g 3-Aminopropionsäure versetzt. Anschließend wurde mit 27 ml 15 gew.-%iger wäßriger Natronlauge ein pH-Wert von 8,5 bis 9,0 eingestellt und 20 Stunden unter Rühren bei diesem Wert gehalten. Es wurde durch Filtration geklärt. Das Filtrat wurde mit 28 gew.-%iger Salzsäure auf einen pH-Wert von 5,0 eingestellt. Die entstandene kristalline Fällung wurde abgesaugt, mit 300 ml Wasser gewaschen und bei 60°C getrocknet. Erhalten wurden 147,2 g einer Verbindung der Formel (I) mit R¹ = H und R² = -CH₂-CH₂-COOH oder der Formel

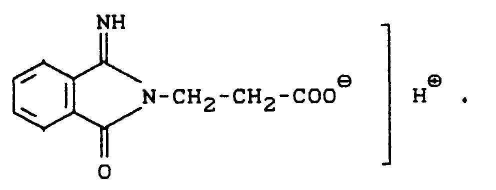

| Elementaranalyse: | C | H | N | O |
|---|---|---|---|---|
| ber.: | 60,6 | 4,6 | 12,8 | 22,0 % |
| gef.: | 60,0 | 4,6 | 12,4 | 22,9 % |

### Beispiel 12

146 g des nach Beispiel 2 erhaltenen 3-Iminoisoindolinons wurden in 700 ml Methanol angerührt und mit 90 g 3-Aminopropanol versetzt. Nach 1-stündigem Rühren bei 45°C wurde erkalten gelassen, abgesaugt, mit 100 ml Methanol gewaschen und im Vakuum bei 60°C getrocknet. Erhalten wurden 109,5 g einer Verbindung der Formel (I) mit R¹ = H und R² = -CH₂-CH₂-CH₂OH.

| Elementaranalyse: | C | H | N | O |
|---|---|---|---|---|
| ber.: | 64,7 | 5,9 | 13,7 | 15,7 % |
| gef.: | 64,7 | 5,8 | 13,7 | 15,8 % |

### Beispiel 13

40,8 g des nach Beispiel 12 erhaltenen 2-Hydroxypropyl-3-imino-isoindolinons wurden unter Kühlung bei 10 bis 20°C in 120 ml Schwefelsäuremonohydrat eingetragen. Nach 3-stündigem Rühren bei Raumtemperatur wurde auf 350 g Eis und 100 ml Wasser ausgetragen. Die kristalline Fällung wurde abgesaugt, mit Eiswasser neutral gewaschen und bei 60°C im Vakuum getrocknet. Es wurden 40,2 g einer Verbindung der Formel (I) mit R¹ = H und R² = -CH₂-CH₂-CH₂-OSO₃H oder der Formel

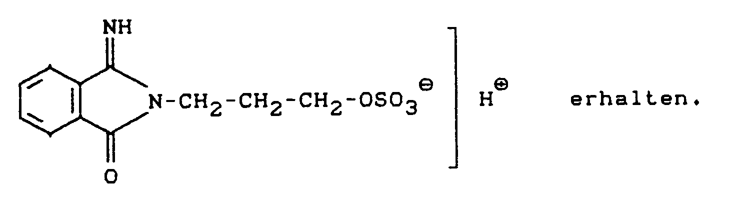

| Elementaranalyse: | C | H | N | O | S |
|---|---|---|---|---|---|
| ber.: | 46,5 | 4,2 | 9,8 | 28,2 | 11,3 % |
| gef.: | 45,9 | 4,4 | 9,8 | 29,1 | 11,2 % |

Schmelzpunkt: 115°C

### Beispiel 14

146 g 1-Iminoisoindolinon wurden in 750 ml Methanol angerührt und mit 106,8 g 2-Amino-1-butanol versetzt. Es wurde 5 Stunden bei 40 bis 45°C und 2 1/2 Stunden bei 60 bis 65°C gerührt und anschließend das Lösungsmittel unter Vakuum im Rotationsverdampfer bei 60°C entfernt. Der Rückstand wurde bei 100°C aufgeschmolzen und dann innerhalb von 2 Stunden in 500 ml Schwefelsäuremonohydrat eingetragen. Nach Zusatz von 150 ml 20 gew.-%igem Oleum wurde 10 Stunden bei Raumtemperatur gerührt. Anschließend wurde auf 400 ml Eiswasser gegeben. Nach 1 Stunde wurde die kristalline Fällung abgesaugt, mit Wasser neutral gewaschen und bei 60°C im Vakuum getrocknet. Erhalten wurden 220,8 g einer Verbindung der Formel (I) mit R¹ = H und R² = -CH(CH₂CH₃)-CH₂-OSO₃H oder der Formel

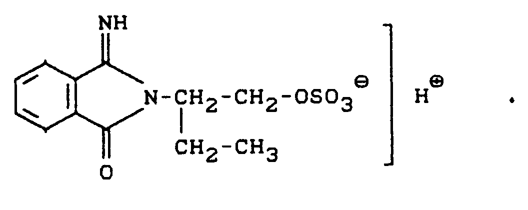

| Elementaranalyse: | C | H | N | O | S |
|---|---|---|---|---|---|
| ber.: | 48,3 | 4,7 | 9,4 | 26,9 | 10,7 % |
| gef.: | 48,5 | 4,8 | 9,0 | 26,3 | 10,5 % |

### Beispiel 15

a) 87,0 g 3-Amino-1-iminoisoindolenin wurden in 600 ml Methanol eingerührt und mit 76,9 g 2-Aminoethanol versetzt. Es wurde langsam zum Sieden erhitzt (Rückfluß). In der anfänglich klaren Lösung trat bald eine kristalline Fällung auf. Nach 10-stündigem Rühren unter Rückfluß wurde auf Raumtemperatur abgekühlt und noch 10 Stunden nachgerührt. Der Niederschlag wurde abgesaugt, mit 200 ml Methanol gewaschen und bei 65°C im Vakuum getrocknet. Erhalten wurden 129,4 g der Verbindung

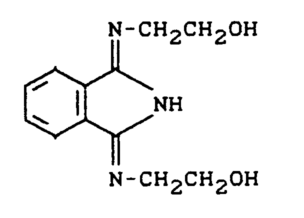

| Elementaranalyse: | C | H | N | O |
|---|---|---|---|---|
| ber.: | 61,8 | 6,5 | 18,0 | 13,7 % |
| gef.: | 61,8 | 6,6 | 17,9 | 13,8 % |

b) 46,6 g der nach a) erhaltenen Verbindung wurden in 230 ml Schwefelsäuremonohydrat eingerührt und anschließend mit 100 ml 20 gew.-%igem Oleum versetzt. Nach 48 Stunden Rühren bei Raumtemperatur wurde das Reaktionsgemisch in 2000 ml Eiswasser eingerührt. Nach 24 Stunden wurde der kristalline Niederschlag abgesaugt, mit Eiswasser neutral gewaschen und bei 60°C im Vakuum getrocknet. Es wurden 43,9 g einer Verbindung der Formel (I) mit R¹ = -CH₂-CH₂-OSO₃H und R² = H oder der Formel 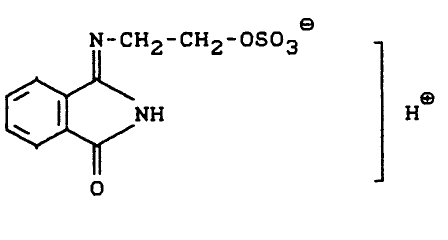 erhalten.

| Elementaranalyse: | C | H | N | O | S |
|---|---|---|---|---|---|
| ber.: | 44,4 | 3,7 | 10,4 | 29,6 | 11,9 % |
| gef.: | 43,2 | 3,8 | 10,5 | 30,6 | 12,0 % |

### Beispiel 16

146,0 g 3-Iminoisoindolinon wurden in 1500 ml Aceton angerührt und mit 173,0 g 85 gew.-%iger Phosphorsäure versetzt. Nach 2-stündigem Rühren wurde abgesaugt, mit 2000 ml Aceton gewaschen und bei 60°C im Vakuum getrocknet. Erhalten wurden 232,0 g einer Verbindung der Formel (II) mit R¹ = R² = H und An^{⊖} = Dihydrogenphosphat.

| Elementaranalyse: | C | H | N | P |
|---|---|---|---|---|
| ber.: | 39,3 | 3,7 | 11,5 | 12,7 % |
| gef.: | 40,3 | 3,7 | 11,7 | 13,8 % |

### Beispiel 17

73,0 g 3-Iminoisoindolinon wurden in 1500 ml Wasser angerührt und anschließend mit 100,5 g 11-Aminoundecansäure versetzt. Es wurde auf 50°C erwärmt und durch Zutropfen von 15 gew.-%iger wäßriger Natronlauge der pH-Wert bei 9,0 bis 9,5 gehalten. Nach 1-stündigem Rühren bei 50°C wurde durch Filtration geklärt und das Filtrat mit 28 gew.-%iger Salzsäure auf einen pH-Wert von 2,0 eingestellt. Es wurde mit 1500 ml Wasser verdünnt, 1 Stunde nachgerührt, abgesaugt und mit Wasser neutral gewaschen. Nach dem Trocknen bei 60°C im Vakuum wurden 150,8 g einer Verbindung der Formel (I) mit R¹ = H und R² = -(CH₂)₁₀COOH oder der Formel 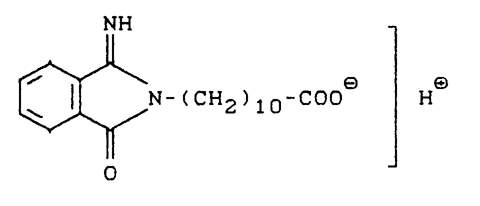
erhalten.

### Beispiel 18 (Tonerbeispiel)

90 g Styrol-n-butylmethacrylat-Copolymer wurden mit 7 g Ruß und 3 g des nach Beispiel 7 erhaltenen Iminoisoindolinonderivats bei 150°C verknetet. Nach Zerkleinerung, grober Vormahlung und anschließender Vermahlung in einer Strahlmühle wurde durch abschließende Sichtung ein Testtoner mit d₅₀ -13 µm isoliert. Die triboelektrische Aufladung gegenüber einem Reineisencarrier betrug -16,7 µC/g und war von der Luftfeuchtigkeit praktisch unbeeinflußt. Im Gerätetest wurde praktisch keine Abnahme der Aufladung festgestellt (-16,5 µC/g nach 70.000 Kopien).

### Beispiel 19 (Tonerbeispiel)

90 g Styrol-2-ethylhexylacrylat-Copolymer wurden mit 7 g Ruß und 3 g des nach Beispiel 13 erhaltenen Iminoisoindolinonderivats bei 150°C verknetet. Nach Zerkleinerung, grober Vormahlung und anschließender Vermahlung in einer Strahlmühle wurde durch abschließende Sichtung ein Testtoner mit d₅₀ -13 µm isoliert. Die triboelektrische Aufladung gegenüber einem Reineisencarrier betrug -13,5 µC/g und war von der Luftfeuchtigkeit praktisch unbeeinflußt. Im Gerätetest wurde keine Abnahme der Aufladung festgestellt (-13,8 µC/g nach 70.000 Kopien).

### Beispiel 20 (Tonerbeispiel)

90 g Styrol-n-butylmethacrylat-Copolymer wurden mit 7 g Ruß, 3 g des nach Beispiel 14 erhaltenen Iminoisoindolinonderivats und 50 g Magnetpigment (Bayferrox® 8610) bei 150°C verknetet. Nach Zerkleinerung, grober Vormahlung und anschließender Vermahlung in einer Strahlmühle wurde durch abschließende Sichtung ein Testtoner mit d₅₀ -13 µm isoliert. Die Aufladung im Gerätetest betrug -8,3 µC/g und war von der Luftfeuchtigkeit unbeeinflußt. Es wurde keine Abnahme der Aufladung von 70.000 Kopien festgestellt.

### Beispiele 21 - 27 (Tonerbeispiele)

Die Herstellung von Tonern erfolgte analog Beispiel 18. Die folgenden triboelektrischen Aufladungen wurden gemessen:

| Beispiel Nr. | Iminoisoindolinon-Derivat aus Beispiel Nr. | Triboelektrische Aufladung [µC/g] |
|---|---|---|
| 21 | 3 | -4,8 |
| 22 | 4 | -3,1 |
| 23 | 6 | -7,5 |
| 24 | 8 | -6,7 |
| 25 | 9 | -5,3 |
| 26 | 11 | -3,1 |
| 27 | 17 | -2,3 |

## Patentansprüche

1. Elektrofotografische Toner, die übliche Harz- und Pigmentpartikel enthalten, dadurch gekennzeichnet, daß sie ein die negative Ladung verstärkendes Additiv der allgemeinen Formeln (I) und/oder (II) enthalten 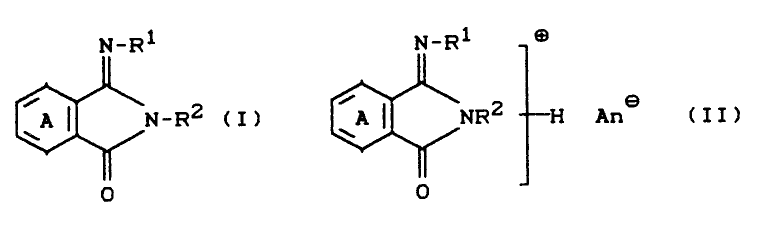 in denen
einer der Reste R¹ und R² für Wasserstoff und der andere dieser Reste für Wasserstoff, C₁-C₂₂-Alkyl, Allyl, Cyclohexyl, Phenyl-C₁-C₂-alkyl oder Phenyl steht,
An^{⊖} ein Anion bedeutet,
der Ring A und/oder die Reste R¹ oder R² substituiert sein können und
der an der Klammer bei Formel (II) stehende Wasserstoff an ein Stickstoffatom gebunden ist,
und/oder eine den Formeln (I) und/oder (II) entsprechende tautomere Verbindung enthalten.

2. Elektrofotografische Toner nach Anspruch 1, dadurch gekennzeichnet, daß in den Formeln (I) und/oder (II) einer der Reste R¹ und R² für Wasserstoff und der andere dieser Reste für Wasserstoff, gegebenenfalls substituiertes C₁-C₁₈-Alkyl, gegebenenfalls substituiertes Benzyl oder gegebenenfalls substituiertes Cyclohexyl steht.

3. Elektrofotografische Toner nach Ansprüchen 1 und 2, dadurch gekennzeichnet, daß in Formel (II) An^{⊖} für Chlorid, Bromid, Iodid, Tetrafluoroborat, das Anion einer Alkylsulfon-, Arylsulfon-, Alkylcarbon-, Arylcarbon-, Alkylphosphon- oder Arylphosphonsäure steht oder ein Bestandteil von R¹ oder R² ist, wenn einer dieser Reste für eine Sulfoalkyl-, Sulfatoalkyl- oder Carboxyalkylgruppe in Form eines Anions steht.

4. Verfahren zur Herstellung von Verbindungen der Formel (II) (siehe Anspruch 1), bei denen sich der anionische Rest am Substituenten R¹ oder R² befindet, dadurch gekennzeichnet, daß man eine Verbindung der Formel (III) 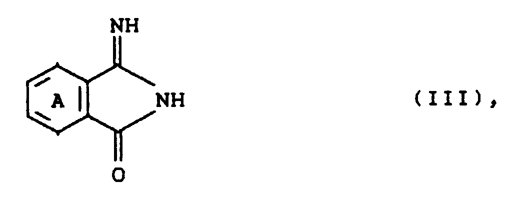 in der der Ring A gegebenenfalls durch C₁-C₄-Alkyl, C₁-C₄-Alkoxy, Hydroxy, Halogen, Cyan, einen Carbamoyl- oder Sulfamoylrest, der gegebenenfalls durch 1 bis 2 C₁-C₄-Alkylreste substituiert sein kann, C₁-C₄-Alkoxycarbonyl oder Phenyl substituiert sein kann, mit einem entsprechend substituierten Amin umsetzt.

5. Verfahren zur Herstellung von Verbindungen der Formel (II) (siehe Anspruch 1), in denen R¹ und R² für Wasserstoff stehen, dadurch gekennzeichnet, daß man ein Isoindolin der Formel (IV) 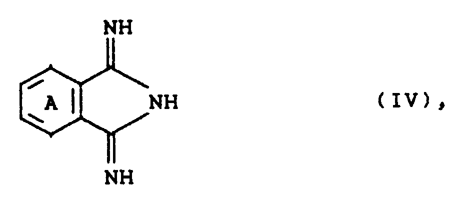 in der der Ring A gegebenenfalls wie bei Formel (III) in Anspruch 4 angegeben substituiert sein kann
mit einer Verbindung der Formel H^{⊕}An^{⊖}, worin An^{⊖} für ein Anion steht, in einem Alkanol umsetzt.

6. Verfahren zur Herstellung von elektrofotografischen Tonern des Anspruchs 1, dadurch gekennzeichnet, daß man 1 bis 3 Gew.-% die negative Ladung verstärkendes Additiv der allgemeinen Formeln (I) und/oder (II) (siehe Anspruch 1), 2 bis 5 Gew.-% Pigmente und gegebenenfalls weitere Additive in ein Basisharz aus Styrol/Acrylat-Copolymer, Styrol/Methacrylat-Copolymer, Styrol/Butadien-Copolymer oder Polyester einknetet, anschließend fein mahlt, nachfolgend eine definierte Teilchengröße durch Sichtung einstellt und gegebenenfalls abschließend feinteilige Kieselsäure hinzufügt.

7. 3-Iminoisoindolinone der Formel (I') 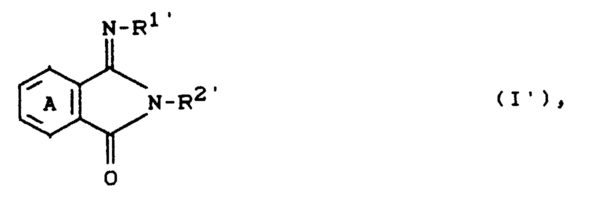 in der
einer der Reste R^{1'} und R^{2'} für Wasserstoff und der andere dieser Reste für Wasserstoff oder eine C₁-C₂₂-Alkylgruppe steht, die durch eine oder mehrere Carboxy-, Sulfo- und/oder Sulfatogruppen substituiert ist.

8. 3-Iminoisoindolinone gemäß Anspruch 7, dadurch gekennzeichnet, daß es sich um
